# EUROPÄISCHE PATENTANMELDUNG

(11) **EP 0 691 323 A1**
(43) Veröffentlichungstag der Anmeldung: **10.01.1996**
(21) Anmeldenummer: 95109578.5
(22) Anmeldetag: 21.06.1995
(51) Int. Cl.: C07C 65/03, C07C 51/363

(54) **Verfahren zur Herstellung von 3,5-Dihydroxy-4-brombenzoesäure**

(30) Priorität: 04.07.1994 DE 4423356
(71) Anmelder: BAYER AG, D-51368 Leverkusen (DE)
(72) Erfinder: Mais, Franz-Josef, Dr., D-40591 Düsseldorf (DE); Fiege, Helmut, Dr., D-51373 Leverkusen (DE); Lehment, Klaus-Friedrich, Dr., D-51519 Odenthal (DE)

(57) **Zusammenfassung**

Mit besonders hohen Ausbeuten und in guten Reinheiten erhält man 3,5-Dihydroxy-4-brombenzoesäure, wenn man 3,5-Dihydroxybenzoesäure in wäßrigen Mineralsäuren bromiert.

## Beschreibung

Die vorliegende Erfindung betrifft ein besonders vorteilhaftes Verfahren zur Herstellung von 3,5-Dihydroxy-4-brombenzoesäure durch Bromierung von 3,5-Dihydroxybenzoesäure.

3,5-Dihydroxy-4-brombenzoesäure ist ein wertvolles Zwischenprodukt, das zur Herstellung von Farbstoffen und Pharmazeutika Verwendung findet (siehe DE-A 24 52 889).

Aus J. Chem. Soc. (C) 1971, 3495 bis 3504 ist es bekannt, die Bromierung von 3,5-Dihydroxybenzoesäure mit elementarem Brom in Gegenwart von Wasser durchzuführen. Nach direkter erschöpfender Methylierung des Reaktionsproduktes zeigt die gaschromatographische Analyse, daß sich praktisch kein 4-Brom-Isomeres gebildet hat. Weiterhin beschreibt diese Literaturstelle, daß die in älterer Literatur (Annalen 164, 109 (1872)) erwähnte Herstellung der 3,5-Dihydroxy-4-brombenzoesäure durch Bromierung von 3,5-Dihydroxybenzoesäure in Wasser nur dann nennenswerte Mengen von 3,5-Dihydroxy-4-brombenzoesäure liefert, wenn der Ansatz nach Abreaktion des Broms auf einem Dampfbad erhitzt wird. Durch erschöpfende Methylierung eines gemäß der o.a. Literaturstelle aus Annalen hergestellten und nachbehandelten Reaktionsgemisches wurde gaschromatographisch gezeigt, daß 73,4 % des gewünschten 4-Brom-Isomeren entstanden waren.

Weiterhin ist die Herstellung von 3,5-Dihydroxy-4-brombenzoesäure durch Bromierung in Essigsäure bekannt (DE-A 26 27 874). Dort ist eine Ausbeute von 92 % angegeben. Wie die Nacharbeitung zeigt, kann man durch Bromieren gemäß der angegebenen Vorschrift (siehe Beispiel 30 der DE-A 26 27 874 und das vorliegende Vergleichsbeispiel 2) diese Ausbeute bei weitem nicht erreichen. Ein nicht unerheblicher Teil des Reaktionsproduktes besteht aus einem in der Mutterlauge gelösten Teer.

Es besteht daher noch immer ein Bedürfnis nach einem technisch realisierbaren Verfahren zur Herstellung von möglichst reiner 3,5-Dihydroxy-4-brombenzoesäure in hohen Ausbeuten.

Es wurde nun ein Verfahren zur Herstellung von 3,5-Dihydroxy-4-brombenzoesäure durch Bromierung von 3,5-Dihydroxybenzoesäure mit elementarem Brom gefunden, das dadurch gekennzeichnet ist, daß man 3,5-Dihydroxybenzoesäure mit wäßriger Mineralsäure mischt und diese Mischung mit Brom versetzt. Gegebenenfalls kann ein Teil des Broms in situ erzeugt werden, z.B. durch Zugabe eines geeigneten Oxidationsmittels.

Vorzugsweise setzt man wäßrige Mineralsäuren ein, die unter den Reaktionsbedingungen nicht mit Brom oder dem Oxidationsmittel reagieren. Folgende Mineralsäuren in wäßriger Form sind beispielsweise geeignet: Salzsäure, Bromwasserstoffsäure, Schwefelsäure, Phosphorsäure und Fluorsulfonsäure. Bevorzugt sind wäßrige Salzsäure, wäßrige Bromwasserstoffsäure und wäßrige Schwefelsäure; besonders bevorzugt ist wäßrige Salzsäure.

Die Konzentration der eingesetzten Mineralsäure kann in einem weiteren Bereich variiert werden. Bei Konzentrationen über beispielsweise 50 Gew.-% beginnt die Löslichkeit des Edukts abzunehmen, und die Reaktionsgeschwindigkeit geht zurück. Sehr kleine Mineralsäurekonzentrationen, z.B. von unter 5 Gew.-%, ergeben Produkte von geringerer Reinheit und lassen die Ausbeute absinken. Die Konzentration der wäßrigen Mineralsäure wird deshalb vorzugsweise im Bereich von 5 bis 50 Gew.-% gewählt. Besonders bevorzugt sind Konzentrationen im Bereich 10 bis 35 Gew.-%. Diese Konzentrationsangaben beziehen sich auf die eingesetzte Menge Wasser plus Mineralsäure.

Auch die Menge der eingesetzten 3,5-Dihydroxybenzoesäure kann in einem weiten Bereich variiert werden. Bei sehr hohen Einsatzmengen sollte vorher überprüft werden, ob das Reaktionsgemisch noch ausreichend rührbar ist. Bezogen auf die eingesetzte Menge Wasser + Mineralsäure kann man beispielsweise 1 bis 75 Gew.-% 3,5-Dihydroxybenzoesäure einsetzen. Vorzugsweise liegt diese Menge im Bereich 10 bis 40 Gew.-%.

Wenn man einen Teil des erforderlichen Broms in situ durch Zugabe eines Oxidationsmittels aus gebildetem Bromwasserstoff herstellen will, so kommen dafür Oxidationsmittel infrage, deren Oxidationspotential größer ist als das des Broms und die mit den organischen Substraten und den Mineralsäuren nicht in unerwünschter Weise reagieren. Beispiele sind Chlor, Wasserstoffperoxid, Sauerstoff, Ozon, Natriumhypochlorit (Chlorbleichlauge) und Peroxodisulfate. Bevorzugt sind Chlor und Wasserstoffperoxid.

Die anzuwendende Brommenge kann im Prinzip ebenfalls in einem weiten Bereich variiert werden. Zur Erzielung einer hohen Ausbeute ist es vorteilhaft, sowohl einen großen Überschuß als auch einen großen Unterschuß an Brom zu vermeiden. Bevorzugt setzt man deshalb 90 bis 110 Mol-% Brom ein, bezogen auf 3,5-Dihydroxybenzoesäure. Besonders bevorzugt beträgt diese Menge 95 bis 105 Mol-%. Das Brom kann als elementares Brom zugefügt werden oder zum Teil in situ aus gebildeter Bromwasserstoffsäure und einem Oxidationsmittel gebildet werden. Gasförmige Oxidationsmittel, z.B. Sauerstoff oder Chlor, kann man z.B. als solche in das Reaktionsgemisch einleiten. Salze oder Wasserstoffperoxid können z.B. als wäßrige Lösung eingesetzt werden.

Soweit das einzusetzende Brom als elementares Brom zugegeben wird, kann es unverdünnt oder verdünnt zum Einsatz gelangen. Als Verdünnungsmittel kommen z.B. wäßrige Mineralsäure oder Wasser in Frage. Bevorzugt ist die Zugabe von unverdünntem Brom.

Bei der Durchführung des erfindungsgemäßen Verfahrens ist es vorteilhaft, nach Beendigung der Zugabe des Broms und gegebenenfalls der Zugabe des Oxidationsmittels bei einer Temperatur von über 50°C, vorzugsweise bei Reaktionstemperatur, nachzurühren. Dieses Nachrühren kann z.B. für 0,5 bis 10 Stunden durchgeführt werden.

Das Reaktionsgemisch kann neben den genannten Reaktionspartnern und der wäßrigen Mineralsäure gegebenenfalls noch weitere Stoffe, z.B. Salze enthalten. Als Beispiele seien genannt: Alkalimetallhalogenide wie Natriumchlorid, Kaliumchlorid, Natriumbromid und Lithiumchlorid, Erdalkalihalogenide wie Magnesiumchlorid und Calciumchlorid und Salze von Übergangsmetallen wie Zinkchlorid und Eisenchlorid. Bei Salzzusätzen läßt sich die hergestellte 3,5-Dihydroxy-4-brombenzoesäure im allgemeinen besser abtrennen.

Das erfindungsgemäße Verfahren kann z.B. in einem Temperaturbereich von Raumtemperatur bis zum Siedepunkt des jeweiligen Reaktionsgemisches durchgeführt werden. Bevorzugt sind Temperaturen im Bereich von 50°C bis zum Siedepunkt des Reaktionsgemisches, besonders bevorzugt im Bereich von 70°C bis zum Siedepunkt des Reaktionsgemisches.

Das erfindungsgemäße Verfahren kann bei Normaldruck, erhöhtem oder erniedrigtem Druck durchgeführt werden. Bevorzugt ist bei Verwendung von Brom und Chlor eine geschlossene Fahrweise bei der sich der Druck entsprechend der Temperatur auf einen leicht erhöhten bzw. erniedrigten Druck selbstständig einstellt.

Eine erste beispielhafte Ausführungsform des erfindungsgemäßen Verfahrens wird wie folgt durchgeführt:
Man legt in einem Reaktionsgefäß wäßrige Mineralsäure und 3,5-Dihydroxybenzoesäure vor, erhitzt unter Rühren auf die gewünschte Reaktionstemperatur, dosiert das erforderliche Brom in elementarer Form ein und rührt einige Stunden bei Reaktionstemperatur nach.

Bei einer anderen beispielhaften Ausführungsform des erfindungsgemäßen Verfahrens legt man ebenfalls wäßrige Mineralsäure und 3,5-Dihydroxybenzoesäure vor, erhitzt unter Rühren auf die gewünschte Reaktionstemperatur, dosiert dann aber nur einen Teil des erforderlichen Broms, nicht weniger als 50 %, in elementarer Form ein. Anschließend erzeugt man das noch benötigte Brom in situ durch Eindosieren eines der genannten Oxidationsmittel und rührt noch einige Stunden bei Reaktionstemperatur nach.

Beim Zusammengeben der wäßrigen Mineralsäure und der 3,5-Dihydroxybenzoesäure kann sich eine Lösung oder eine Suspension ergeben.

Es ist ausgesprochen überraschend, daß beim erfindungsgemäßen Verfahren der Wechsel des zur Reaktion eingesetzten Mediums zu einer deutlich erhöhten Ausbeute führt und 3,5-Dihydroxy-4-brombenzoesäure in höherer Reinheit erhalten wird. Dies ist besonders deshalb überraschend, weil auch gemäß dem Stand der Technik im Laufe der Reaktion Bromwasserstoff gebildet wird, der die Acidität des Mediums ansteigen läßt. Durch den glatten Verlauf der erfindungsgemäßen Reaktion können gewünschtenfalls die in den Mutterlaugen vorhandenen Reststoffe zurückgewonnen und gegebenenfalls in den Produktionsprozeß zurückgeführt werden.

Die nachfolgenden Beispiele erläutern das erfindungsgemäße Verfahren.

### Beispiel 1

500 g 3,5-Dihydroxybenzoesäure und 1500 ml 18 gew.-%ige wäßrige Salzsäure wurden zusammengegeben, auf schwachen Rückfluß (ca. 106°C Innentemperatur) erhitzt und gerührt. Dann wurden 518 g Brom im Verlaufe von 2,5 Stunden mit gleichmäßiger Geschwindigkeit zudosiert. Danach wurde noch 3 Stunden am leichten Rückfluß (107 bis 108°C) nachgerührt. Anschließend wurde unter Rühren auf Raumtemperatur abgekühlt, das ausgefallene Pulver abfiltriert und getrocknet.

Auswaage: 735,7 g fast farbloses Pulver, Reinheit (gemäß HPLC): 96,9 % 3,5-Dihydroxy-4-brombenzoesäure, Ausbeute: 94,2 %, bezogen auf eingesetzte 3,5-Dihydroxybenzoesäure.

Die Einengung der Mutterlauge lieferte nach dem Trocknen weitere 19,7 g eines Feststoffs (hellbraunes Pulver).

### Vergleichsbeispiel 1

Es wurde analog Beispiel 1 verfahren, jedoch statt wäßriger Salzsäure die gleiche Menge Wasser eingesetzt. Die Rückflußtemperatur betrug beim Beginn der Zudosierung von Brom 100°C und während der 3-stündigen Nachrührzeit 101 bis 103°C.

Es wurden 440,5 g eines hellbeigen Pulvers isoliert, dessen Reinheit (gemäß HPLC) 90,2 % betrug. Dies entspricht einer Ausbeute von 52,5 % an 3,5-Dihydroxy-4-brombenzoesäure.

Die Einengung der Mutterlauge lieferte weitere 314,2 g eines beigefarbenen Pulvers mit einem Gehalt an 3,5-Dihydroxy-4-brombenzoesäure (HPLC) von 78,8 %. Das entspricht weiteren 32,7 % Ausbeute.

### Vergleichsbeispiel 2

50 g 3,5-Dihydroxybenzoesäure und 180 ml Eisessig wurden zusammengegeben, bis zum Rückfluß (115°C) erhitzt und gerührt. Dann wurden 54,0 g Brom in Verlaufe von 3 Stunden langsam eindosiert, danach noch 1 Stunde am Rückfluß (111°C) nachgerührt. Nach dem Abkühlen auf 10°C wurde abgesaugt und das isolierte Festprodukt im Vakuum getrocknet. Man erhielt 70,2 g 3,5-Dihydroxy-4-brombenzoesäure in Form eines rosafarbenen Feststoffes, dessen Reinheit 84,1 % betrug. Das entspricht einer Ausbeute von 78,0 %.

Die Mutterlauge wurde eingeengt und so weitere 15,0 g eines schwarzbraunen, in der Kalte glasigen Teers erhalten.

### Beispiel 2

Es wurde analog Beispiel 1 gearbeitet, jedoch statt der wäßrigen Salzsäure die gleiche Menge 24 gew.-%iger wäßriger Bromwasserstoffsäure eingesetzt.

Es wurden 725,8 g 3,5-Dihydroxy-4-brombenzoesäure mit einer Reinheit von 96,0 % erhalten. Das entspricht einer Ausbeute von 92,0 %.

### Beispiel 3

Es wurde analog Beispiel 1 gearbeitet, jedoch statt der wäßrigen Salzsäure die gleiche Menge 20 gew.-%iger wäßriger Schwefelsäure eingesetzt.

Es wurden 696,3 g 3,5-Dihydroxy-4-brombenzoesäure mit einer Reinheit von 95,2 % erhalten. Das entspricht einer Ausbeute von 87,5 %.

### Beispiel 4

308 g 3,5-Dihydroxybenzoesäure wurden in 924 g 17,5 gew.-%iger wäßriger Salzsäure eingerührt und auf 104°C unter Rühren erhitzt. Dann wurden im Verlauf von 1,5 Stunden 192 g Brom eindosiert. Direkt anschließend wurden bei der gleichen Temperatur 78 g 35 gew.-%ige wäßrige Wasserstoffperoxidlösung innerhalb von 2 Stunden eindosiert. Nachdem noch 4 Stunden am Rückfluß (ca. 107°C) nachgerührt wurde, wurde auf Raumtemperatur abgekühlt, das angefallene Pulver abgesaugt und getrocknet. Auswage: 450,3 g hellbraunes Pulver, Reinheit (gemäß HPLC): 95,7 % 3,5-Dihydroxy-4-brombenzoesäure, Ausbeute: 92,4 %, bezogen auf eingesetzte 3,5-Dihydroxybenzoesäure.

### Beispiel 5

Es wurde analog Beispiel 4 verfahren, jedoch wurde anstelle von wäßrigem Wasserstoffperoxid 60,0 g Chlor eindosiert.

Es wurden 433,8 g 3,5-Dihydroxy-4-brombenzoesäure mit einer Reinheit von 94,0 % (HPLC) erhalten. Das entspricht einer Ausbeute von 87,5 %, bezogen auf eingesetzte 3,5-Dihydroxybenzoesäure.

## Patentansprüche

1. Verfahren zur Herstellung von 3,5-Dihydroxy-4-brombenzoesäure durch Bromierung von 3,5-Dihydroxybenzoesäure mit elementarem Brom, dadurch gekennzeichnet, daß man 3,5-Dihydroxybenzoesäure mit wäßriger Mineralsäure mischt und diese Mischung mit Brom versetzt.

2. Verfahren nach Anspruch 1, dadurch gekennzeichnet, daß man einen Teil des Broms in situ durch Zugabe eines Oxidationsmittels erzeugt.

3. Verfahren nach Ansprüchen 1 und 2, dadurch gekennzeichnet, daß man als wäßrige Mineralsäure Salzsäure, Bromwasserstoffsäure, Schwefelsäure, Phosphorsäure oder Fluorsulfonsäure in wäßriger Form einsetzt.

4. Verfahren nach Ansprüchen 1 bis 3, dadurch gekennzeichnet, daß die eingesetzte wäßrige Mineralsäure eine Konzentration im Bereich von 5 bis 50 Gew.-%, bezogen auf die eingesetzte Menge Wasser + Mineralsäure, aufweist.

5. Verfahren nach Ansprüchen 1 bis 4, dadurch gekennzeichnet, daß Brom in einer Menge von 90 bis 110 Mol-%, bezogen auf 3,5-Dihydroxybenzoesäure, eingesetzt oder eingesetzt und in situ erzeugt wird.

6. Verfahren nach Ansprüchen 1 bis 5, dadurch gekennzeichnet, daß nach Beendigung der Zugabe des Broms und gegebenenfalls des Oxidationsmittels bei einer Temperatur über 50°C nachgerührt wird.

7. Verfahren nach Ansprüchen 1 bis 6, dadurch gekennzeichnet, daß man es bei Temperaturen zwischen Raumtemperatur und dem Siedepunkt des jeweiligen Reaktionsgemisches durchführt.

8. Verfahren nach Anspruch 2, dadurch gekennzeichnet, daß man als Oxidationsmittel Chlor, Wasserstoffperoxid, Sauerstoff, Ozon, Natriumhypochlorit oder Peroxodisulfate einsetzt.

9. Verfahren nach Ansprüchen 2 und 8, dadurch gekennzeichnet, daß man 50 % oder mehr des erforderlichen Broms in elementarer Form dosiert und das restliche Brom in situ durch Eindosieren eines Oxidationsmittels erzeugt.

10. Verfahren nach Ansprüchen 1 bis 9, dadurch gekennzeichnet, daß man 1 bis 75 Gew.-% 3,5-Dihydroxybenzoesäure, bezogen auf Wasser + Mineralsäure, einsetzt.
